# EUROPEAN PATENT APPLICATION

(11) **EP 4 813 303 A1**
(43) Date of publication of application: **30.09.2026**
(21) Application number: 25165940.5
(22) Date of filing: 25.03.2025
(51) Int. Cl.: A61B 6/00, A61B 6/58, G06T 7/33, A61B 6/06

(54) **METHOD FOR ADJUSTING COMPONENTS OF AN IMAGING SYSTEM**

(71) Applicant: Koninklijke Philips N.V., 5656 AE Eindhoven (NL)
(72) Inventor: MAY, Jan Marek, Eindhoven (NL); FEIERABEND, Volker, Eindhoven (NL); SCHLUETER, Mathias, 5656AG Eindhoven (NL); MENSER, Bernd, Eindhoven (NL)
(74) Representative: Philips Intellectual Property & Standards

(57) **Abstract**

The subject-matter of the present disclosure relates to a computer implemented method for controlling a radiographic imaging system comprising determining (310) a first corrective transformation corresponding to a first configuration of the radiographic imaging system by: (i) obtaining (312) a first set of coordinates of reference points based on a known geometry, (ii) capturing (314) an image comprising the reference points, (iii) obtaining (316) a second set of coordinates of the reference points based on image analysis of the captured image, (iv) determining (318) the corrective transformation by minimising a reprojection error between the first set of coordinates and the second set of coordinates; and determining (320) a second corrective transformation by repeating steps (i) to (iv) for a second configuration. The corrective transformations are for changing a system parameter of one or more components of the radiographic imaging system when in the first configuration or the second configuration.

## Description

### FIELD OF THE INVENTION

The subject-matter of the present disclosure relates to a computer implemented method for adjusting components of an imaging system, and a radiographic imaging system therefor.

### BACKGROUND OF THE INVENTION

Radiographic imaging systems, such as diagnostic systems and interventional systems, include various components that can be moved relative to each other and their environment. To ensure the imaging of such a system is of a suitable quality, it is important to ensure that components are suitably aligned. The components of the system can be arranged in various configurations, which are defined by system geometry parameters.

The system geometry parameters are typically determined via sensors which measure imager position/height, tube height, tube tilt, collimation parameter, and other position and/or orientation information. In some instances, when using these system geometry parameters, an ideal environment is assumed, e.g., an environment with perfectly flat walls, ceiling and floor, each precisely perpendicular to each other. In other instances, an installer can provide correction terms for deviations from the ideal environment, e.g., a correction term for a sloped ceiling. This typically requires the installer to be in the environment, is time-consuming and complicates the installation process. Furthermore, the number of places in which a correction term can be included is very limited, also leading to limited accuracy.

Therefore, despite the use of correction terms, the alignment of the components of the system is typically not accurate in all configurations. Furthermore, measurement errors of the sensors and correction terms are propagated and can further contribute to an inaccurate positioning.

It is an aim of the subject-matter of the present disclosure to improve on the prior art.

### SUMMARY OF THE INVENTION

According to a first aspect of the present invention, there is provided a computer implemented method for controlling a radiographic imaging system, wherein the radiographic imaging system comprises an image sensor, the method comprising: determining a first corrective transformation corresponding to a first configuration of the radiographic imaging system by: (i) obtaining a first set of coordinates corresponding to a set of reference points based on a known geometry of the radiographic imaging system with respect to the set of reference points; (ii) capturing, by the image sensor, an image comprising the set of reference points; (iii) obtaining a second set of coordinates corresponding to the set of reference points based on image analysis of the captured image; (iv) determining the corrective transformation by minimising a reprojection error between the first set of coordinates and the second set of coordinates; and determining a second corrective transformation corresponding to a second configuration of the radiographic imaging system by repeating steps (i) to (iv) for the second configuration, and wherein the first corrective transformation and the second corrective transformation are for changing a system parameter of one or more components of the radiographic imaging system when in the first configuration or the second configuration.

In an example, the method further comprises: controlling a movement of one or more components of the radiographic imaging system between the first configuration and the second configuration based on the first corrective transformation and the second corrective transformation.

In an example, the method further comprises: repeating steps (i) to (iv) for a plurality of configurations of the radiographic imaging system, to determine a plurality of corresponding corrective transformations for the plurality of configurations; and generating a calibration database comprising the configurations and the corrective transformations.

In an example, the method further comprises: receiving an instruction to change the radiographic imaging system from a current configuration to a new configuration; determining a corrective transformation corresponding to the new configuration based on the calibration database.

In an example, when the new configuration is not included in the calibration database, the determining the corrective transformation comprises: determining a closest configuration to the new configuration comprised in the calibration database; and selecting a corresponding closest corrective transformation as the corrective transformation.

In an example, when the new configuration is not included in the calibration database, the determining the corrective transformation comprises: determining a first closest configuration to the new configuration comprised in the calibration database; determining a second closest configuration to the new configuration comprised in the calibration database; and interpolating between the corrective transformations corresponding to the first closest configuration and the second closest configuration comprised in the database; and generating the corrective transformation based on the interpolation.

In an example, step (iii) comprises inputting the captured image to a trained machine learning model.

In an example, step (ii) comprises capturing a sequence of images, and step (iv) comprises minimising the reprojection error between the first set of coordinates and the second set of coordinates based on bundle adjustment performed on the sequence of images.

In an example, the image sensor is a second image sensor, and the plurality of reference points are provided on a first image sensor configured to generate a radiographic image.

In an example, the plurality of reference points comprises at least one of: a visually distinguishable point of the first image sensor; and a marker attached to the first image sensor.

In an example, the image sensor is further configured to determine depth in a captured image, and the second set of coordinates of the set of reference points comprises three-dimensional position information.

In an example, the corrective transformation comprises a transformation applied to at least one parameter representing: an offset parameter of a tube head of the radiographic imaging system and a first image sensor configured to generate a radiographic image; a rotation parameter of the tube head; and a collimator opening parameter of a collimator of the radiographic imaging system.

In a related aspect of the invention there is provided a radiographic imaging system comprising a controller configured to carry out the method of any preceding claim.

It will be appreciated that the invention also extends to radiographic imaging systems corresponding to the method aspects and examples outlined above.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows a schematic of a radiographic imaging system;
Fig. 2 shows an example image including exemplary reference points;
Fig. 3 shows a method for controlling the radiographic imaging system of Fig. 1;
Fig. 4 shows a method for determining a corrective transformation; and
Fig. 5 shows an example of repositioning a radiographic imaging system.

### DETAILED DESCRIPTION OF EMBODIMENTS

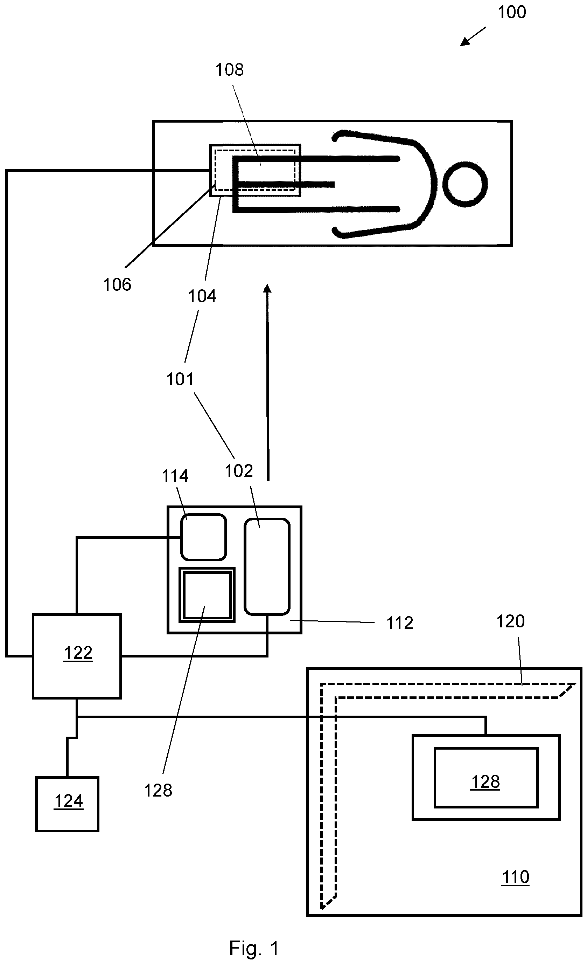

With reference to Fig. 1, there is shown an example radiographic imaging system 100. The radiographic imaging system 100 is based around the emission of ionising radiation and the subsequent imaging of that radiation. For example, the radiographic imaging system 100 may be embodied as an X-ray imaging system, a computed tomography (CT) scan system, a positron emission tomography (PET) scan system, and other variants of imaging systems using ionizing radiation.

The radiographic imaging system 100 is primarily described as a diagnostic system; however, as will be familiar to those in the art, the system 100 could instead be an interventional system.

Suitably, the system 100 comprises a radiographic imaging device 101; for example, comprising an ionising radiation generator/emitter 102 and a radiographic image sensor 104, also termed a first image sensor herein. For example, in the X-ray imaging system, the radiation generator 102 is configured to generate X-rays and the first image sensor 104 is correspondingly configured to capture an X-ray image. If the generator 102 is configured to generate a different form of ionising radiation, the first image sensor 104 is configured accordingly. As will be familiar to those in the art, the arrangement of the radiographic imaging means 102, 104 defines a target area 106 in which a target 108, such as a patient, or part thereof, can be positioned in order to capture a radiographic image based on the penetration of radiation through the target 108 in the target area 106. Put another way, the first image sensor 104 is configured to capture a radiographic image of the target area 106. In some examples, the first image sensor 104 is provided as a separate unit configured to communicate, preferably wirelessly, with other components of the system 100.

In some examples, radiation generator 102 and radiographic image sensor 104 are provided in a fixed arrangement, with the target 108 being moved with respect to the generator 102 and sensor 104 to allow for imaging of different parts of the target 108. Accordingly, in these examples, the target area 106 to be imaged is fixed in position.

In other examples, the radiation generator 102 and radiographic image sensor 104 are coupled to suitable movement means such that one or both of the first image sensor 104 and radiation generator 102 may be moved relative to the target 108. That is, in these examples, the target 108 is substantially fixed in position, while the target area 106 to be imaged may be re-positioned according to the moveable arrangement between the first image sensor 104 and radiation generator 102.

In one example of moveable radiographic imaging means 102, 104, the first image sensor 104 is coupled to one or more rails to provide lateral motion, while the generator 102 is coupled to a moveable arm.

In another example of moveable radiographic imaging means 102, 104, the radiation generator 102 is provided on a suitably moveable head part, or tube head, 112. Meanwhile, the first image sensor 104 is provided as a separate unit configured to communicate wirelessly with other components of the system 100. To capture an image of the target 108, the tube head 112 is aligned with the target 108 and the first image sensor 104 freely positioned behind the target 108 to capture an image of the ionising radiation.

In the example shown in Fig. 1, the system 100 comprises a control station 110, from which control parameters and functions of the radiographic imaging device 101 may be set. The control station 110 is disposed separated from the target area 106 of the radiographic imaging device 101 so as to limit the potential for exposure of the operator of the system 100 to radiation. In some examples, the control station 110 is shielded from the imaging device 101 (more specifically, the ionising radiation generator 102 and target area 106 therefor) by a radiation shield 120.

In some examples, the system 100 comprises a display 128 for viewing images captured by the radiographic imaging device 101. As shown in Fig. 1, in some examples, the display 128 may be provided at the control station 110, or on the tube head 112.

The system 100 comprises an image sensor 114, also called a second image sensor 114 herein, having a field of view which encompasses the target area 106. That is, the second image sensor 114 can capture images of the target 108 within the target area 106. The operator can use the second image sensor 114 to instruct adjustment of the target 108 and/or system 100 in order to allow for correct imaging of the target 108.

The second image sensor 114 may be configured to capture light at any suitable wavelength that allows for positioning the target 108. In one example, the second image sensor 114 is configured to capture visible light; suitably, in such examples, the second image sensor 114 may be embodied as an RGB camera. In another example, the second image sensor 114 is configured to capture infra-red light, and may therefore be embodied as an infra-red camera. In yet another example, the second image sensor 114 is configured to capture depth information; suitably, in such examples the second image sensor 114 may be part of an RGB depth (RGB-D) camera.

In some examples, the second image sensor 114 is integrated as part of the radiographic imaging device 101. That is, as a component of the respective part of the imaging device 101, such as the X-ray generator 102, or a related component thereof. In the present example, the second image sensor 114 is integrated as part of the tube head 112 of the system 100, such that its position substantially corresponds to the position of the radiation generator 102, and its field of view substantially corresponds with the direction of radiation emission from the generator 102, allowing the second image sensor 114 to capture an image of the target area 106.

The system 100 comprises a controller 122 (e.g., a processor) configured to control an operation of the system 100. For example, the controller 122 may be configured to control a data acquisition comprising controlling the radiographic imaging device to capture a radiographic image of the target area 106. That is, the controller 122 controls the radiation generator 102 to emit radiation and the first image sensor 104 to record an image based on the received radiation. Suitably, at substantially the same time, the controller 122 controls the second image sensor 114 to also capture a positional image of the target area 106. That is, the data acquisition also comprises capturing second image sensor 114 data. The captured second image sensor 114 data may be an image, a video, or the like.

Suitably, the data acquisition may be stored in a memory 124. In some examples, the memory 124 is local to the system 100, e.g., an internal memory storage. In other examples, the memory 124 is external to the system 100. In the case of an external memory, the system 100 may comprise suitable transceiver circuitry for wireless communication with the external memory 124.

A data acquisition may be performed with or without a target 108. When a target is present, it may be considered that the system 100 is being operated in a diagnosis mode, and aspects of the system 100 configured and operated accordingly. Conversely, when a target 108 is not present, it may be considered that the system is being operated in a calibration mode, and again various aspects of the system 100 configured and operated accordingly.

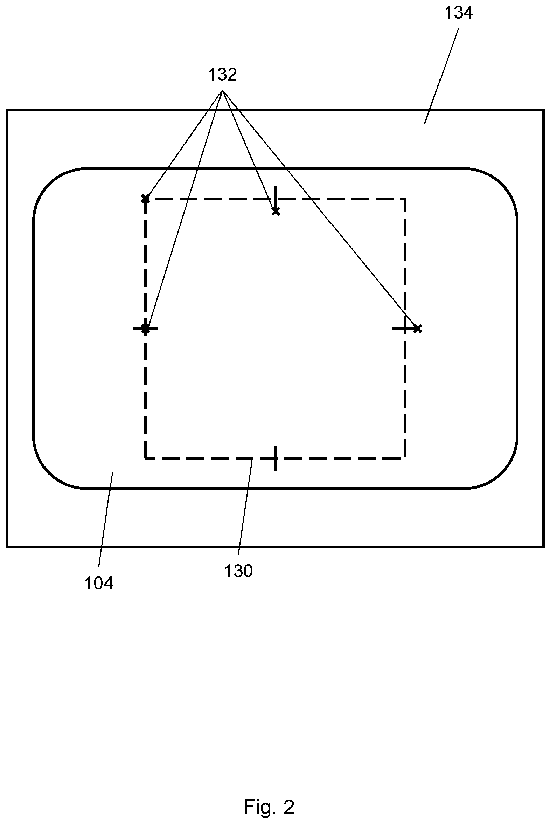

With reference to Fig. 2, there is shown an example of an image 134, i.e., the captured second image sensor 114 data, when the second image sensor 114 is configured to capture an image of the first image sensor 104. As shown, the first image sensor 104 comprises a guide pattern 130 indicating the target area 106 for assisting alignment of the components of the system 100. The image 134 can be displayed on the display 128 during data acquisition for calibration of the system 100, for example, during collimation.

It will be appreciated however that the data acquisition may not result in a suitable radiographic image of the target 108. In some configurations, the expected (i.e., nominal or theoretical) target area, i.e., the area that is expected to be captured by the first image sensor 104 when the first image sensor 104 captures an image, may not align with the target area 106 as indicated by the guide pattern 130. This can lead to problems with the quality of the radiographic image of the target 108, for example, if the wrong part of the target 108 is imaged or if there is excessive radiation scattering. The radiographic imaging system configuration represents the relative positions and orientations of the components of the system, e.g., the tube head 112, the first image sensor 104 and the collimator shutters.

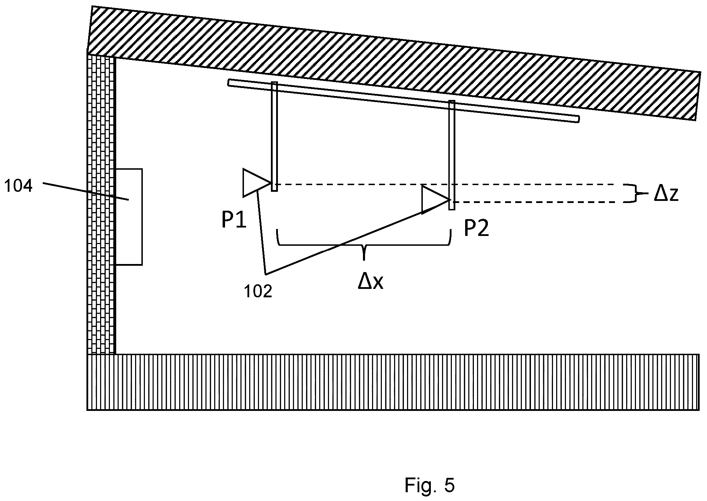

The problem may be exacerbated when moving parts of the system 100, e.g., a position of the radiation generator/emitter 102 from a first position P1 to a second position P2, requires non-planar motion as demonstrated in Fig. 5. Such geometrical changes in position require a less than straight forward readjustment of imaging parameters to yield a desired radiographic result.

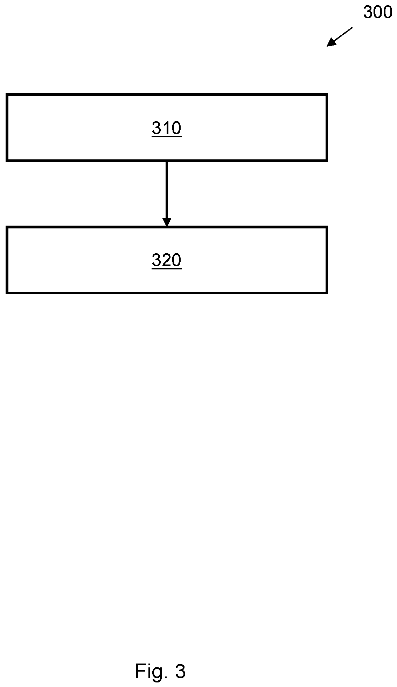

With reference to Fig. 3, to improve the accuracy and consistency of the data acquisition, a computer implemented method 300 for controlling a radiographic imaging system 100 is shown. Corrective transformations for changing a system parameter of one or more components of the system 100 are determined and applied to the system parameter of the component(s).

As shown in Fig. 3, this method includes determining a first corrective transformation corresponding to a first configuration of the radiographic imaging system 310, and determining a second corrective transformation corresponding to a second configuration of the radiographic imaging system 320. The first corrective transformation and the second corrective transformation are for changing a system parameter of one or more components of the radiographic imaging system when in the first configuration or the second configuration, respectively.

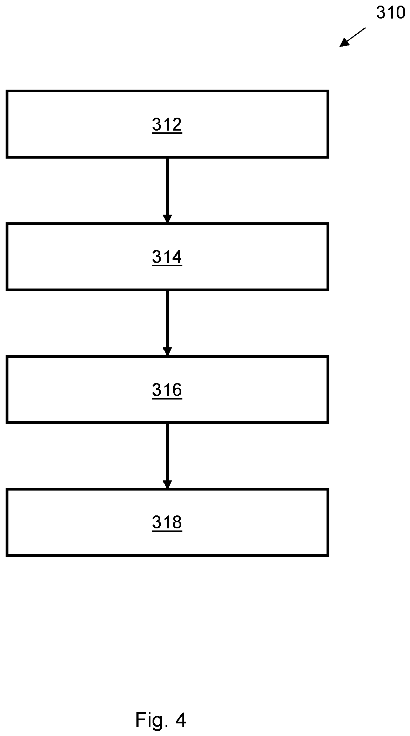

With reference to Fig. 4, determining the first corrective transformation 310 comprises: obtaining a first set of coordinates corresponding to a set of reference points based on a known geometry of the radiographic imaging system with respect to the set of reference points 312; capturing, by the second image sensor, an image comprising the set of reference points 314; obtaining a second set of coordinates corresponding to the set of reference points based on image analysis of the captured image 316; and determining the corrective transformation by minimising a reprojection error between the first set of coordinates and the second set of coordinates 318.

Example reference points 132 are shown in Fig. 2. In an example, the reference points 132 are provided on the first image sensor 104 (i.e., the radiographic image sensor configured to generate a radiographic image) and an image of the first image sensor 104 can be captured by the second image sensor 114 (i.e., the camera). For example, the reference points 132 may include visually distinguishable points of the first image 104 sensor and/or markers attached to the first image sensor 104. Visually distinguishable points of the first image sensor 104 include, for example, corners, edges and centre points of lines and/or areas of the first image sensor 104 itself or the guide pattern 130 thereon. Visually distinguishable points of the first image sensor 104 may also include, for example, pen markings and the like. Markers attached to the first image sensor 104 may include, for example, stickers or other such markings attached to the first image sensor 104.

Returning to Fig. 4, step 312 comprises, while the system is in the first configuration, obtaining the first set of coordinates corresponding to the reference points 132.

In the first configuration, the system 100 has a known geometry, for example, as measured or configured during system installation or as measured or configured once the system 100 is arranged into the first configuration. This known geometry is represented by the system geometry parameters, which define the position(s) and orientation(s) of the system components with respect to each other, and/or with respect to the environment. For example, the system geometry parameters may include a distance between the tube head 112 and the first image sensor 104, an angle between the tube head 112 and the first image sensor 104, the relative positions of the second image sensor 114 and the radiation generator 102 with respect to each other and the tube head 112, the position and orientation of the collimator shutters, and the like.

In the first configuration, there is a first reference frame, i.e., a first coordinate system, that corresponds to the physical system 100 or its environment. In the first configuration, there is also a second reference frame, i.e., a second coordinate system, that corresponds to the second image sensor 114.

Step 312 comprises obtaining coordinates of the reference points 132 in the first reference frame (i.e., the first set of coordinates in the first coordinate system). The first set of coordinates of the reference points 132 are obtained based on the system geometry parameters of the radiographic imaging system 100.

To arrange the system 100 into the first configuration, the radiation beam is arranged to align with the target area 106. This may involve adjusting a system parameter associated with the collimation. In some examples, this is done by using guidance from the second image sensor 114. For example, the image 134 shown on the display 128 may be used to align the radiation beam with the target area 106. In another example, the radiation beam is arranged to align with the target area 106 based on an automatic analysis of the image 134, e.g., by inputting the image 134 into a trained machine learning model.

Step 314 comprises, while the system is in the first configuration, capturing an image comprising the set of reference points 132 by the image sensor. For example, the image 134 is captured by the second image sensor 114, and at least one reference point 132 is included in the image 134.

The image 134 is captured by the second image sensor 114 in the data acquisition, as described hereinbefore, in the first configuration. The image 134 is a positional image of the target area 106 without a target 108 present.

In some examples, step 314 further comprises capturing a sequence of images including at least one reference point 132.

Step 316 comprises, while the system is in the first configuration, obtaining a second set of coordinates corresponding to the set of reference points 132 based on image analysis of the captured image 134.That is, step 316 comprises obtaining coordinates of the reference points 132 in the second reference frame (i.e., the second set of coordinates in the second coordinate system). The second set of coordinates of the reference points 132 are obtained based on image analysis of the image 134 captured by the second image sensor 114.

In some examples, the image analysis of the captured image 134 comprises recognising a reference point 132, and identifying the corresponding coordinate of the reference point 132 in the second image sensor coordinate system. The image analysis can be performed using standard image processing methods, such as edge detection, machine learning techniques, and the like. In some examples, image analysis of the captured image comprises inputting the captured image to a trained machine learning model. In some examples, the reference point coordinates may be identified from a corner, edge and/or centre point of a line and/or area of the first image sensor 104 or the guide pattern 130 thereon, as recognised in the image 134.

In examples in which the second image sensor 114 is configured to capture depth information or determine depth in a captured image, for example, as part of an RGB depth (RGB-D) camera, the second set of coordinates of the set of reference points 132 comprises three-dimensional position information.

Step 318 comprises, while the system is in the first configuration, determining the corrective transformation by minimising a reprojection error between the first set of coordinates and the second set of coordinates. The reprojection error is the geometric error - i.e., the error due to issues with the radiographic imaging system's geometry, such as misalignment of the radiographic imaging system's components - corresponding to the distance in a captured image between a projected point and a measured point. In effect, the first set of coordinates of the reference points 132 is projected from the first reference frame into the second reference frame, so that the projected first set of coordinates are in the second coordinate system. Thus, the projected first set of coordinates can be compared to the second set of coordinates in the second reference frame, i.e., the second coordinate system. In other words, the projected first set of coordinates are the projected points, and the second set of coordinates are the measured points for the reprojection error. In some examples, step 318 further comprises identifying the projected point from the projected first set of coordinates that corresponds to a measured point from the second set of coordinates, or vice versa.

The distance in the second reference frame between the corresponding projected point from the projected first set of coordinates and the measured point from the second set of coordinates is minimised using any suitable minimisation. For example, an Iterative Closest Point (ICP) method can be used to minimise the difference between the projected first set of coordinates and the second set of coordinates.

In some examples, step 314 comprises capturing a sequence of images comprising the set of reference points 132 by the image sensor. For example, a sequence of images is captured by the second image sensor 114. In this case, step 318 may comprise minimising the reprojection error between the first set of coordinates and the second set of coordinates based on bundle adjustment performed on the sequence of images.

Returning to Fig. 3, step 320 comprises determining a second corrective transformation corresponding to a second configuration of the radiographic imaging system. In effect, the system 100 is arranged into a second configuration and steps 312, 314, 316 and 318 are repeated for the second configuration.

To further elucidate the method described herein, the following exemplary method and associated calculations are described.

Herein, ${x}_{w} = \left(x, y, z\right) ∈ {ℝ}^{3}$ denotes a 3-dimensional world (i.e., environment) coordinate in the first coordinate system, and ${x}_{cam} ∈ {ℝ}^{3}$ denotes the 3-dimensional coordinate in the second coordinate system (e.g., the coordinate system of the second image sensor, i.e., the camera). The corresponding homogeneous counterparts, i.e., the corresponding projective coordinates, are denoted by ${\dot{x}}_{w} , {\dot{x}}_{cam} ∈ {ℙ}^{3}$. Hence, a reference point 132 in the second coordinate system ${x}_{cam , n} ∈ {ℝ}^{3}$, where *n* = 0, ... *, N* can be expressed as: ${\dot{x}}_{cam , n , m} = {T}_{w \to cam , m} {\dot{x}}_{w , n} = \left[\begin{matrix}{R}_{w \to cam , m} \\ 0\end{matrix}\left|\begin{matrix}{t}_{w \to cam , m} \\ 1\end{matrix}\right.\right] {\dot{x}}_{w , n} ,$

Where ${T}_{w \to cam , m} ∈ {ℝ}^{4 \times 4}$ denotes the homogeneous transformation consisting of the rotation matrix **R**_{w→cam,*m*} and the translation **t**_{w→cam,*m*} of the second image sensor relative to the first coordinate system coordinates at configuration *m*, where *m* is the index of the current system configuration. For example, in the first configuration, *m* = 1. Therefore, **x**_{cam,*n*,*m*} represents the reference point *n* at system configuration *m* in the second coordinate system.

As the position of the reference points **x**_{w,*n*} is known from the system's known geometry, as described herein, Equation 1 can be used to define its position in the second coordinate system according to the system geometry parameters for the current configuration, when the reference point 132 is located on the first image sensor 104. This transformation determined from the system geometry parameters is denoted by **T̂**_{w→cam,*m*}. When the reference points 132 are visible in the image 134 captured by the second image sensor 114, the projection of the reference points 132 into the camera image can be calculated by: $\left(\begin{matrix}{x}_{c , n} \\ {y}_{c , n} \\ {z}_{c , n}\end{matrix}\right) = K \left[{\hat{R}}_{w \to cam , m}\left|{\hat{t}}_{w \to cam , m}\right.\right] {\dot{x}}_{w , n} ,$ where $K = \left(\begin{matrix}{α}_{x} & s & {x}_{0} \\ 0 & {α}_{y} & {y}_{0} \\ 0 & 0 & 1\end{matrix}\right)$ are the intrinsic parameters of the second image sensor 114.

As the rotation matrix **R̂**_{w→cam,*m*} as well as the translation vector **t̂**_{w→cam,*m*} depend on the accuracy of the sensors for sensing the system geometry and the system 100 setup, the calculated projection of the reference points 132 into the second coordinate system will not overlay the detected location **x**_{ref,*n*,*m*} of the reference point **x***ₙ* in the captured image 134, in configuration m. Furthermore, the intrinsic parameters of the second image sensor 114 depend on the accuracy of the second image sensor calibration procedure and will affect the accuracy of the calculated projected reference point.

In an example, the system components (e.g., the tube head and the first image sensor) are moved to plurality of configurations for calibration. For example, the system components can be moved automatically or manually. For example, the system components can be moved to pre-defined positions and tube head 112 orientations. Ideally, the configurations for calibration cover well the space of possible system configurations that are typical in the use of the radiographic imaging system 100. In the plurality of configurations, a plurality of images are captured by the second image sensor 114.

In one example, the captured images are processed by a keypoint detector which identifies the image locations **x**_{ref,*n*,*m*} of the reference points. Neural networks or other machine learning methods can be used to identify the reference points 132 in the images 134. Alternatively, classical image analysis can be used, for example, if the structure of the reference points 132 is geometrically simple.

In one example, to determine the second image sensor 114 positions and orientations, i.e., poses, from a sequence of images with a different position and/or orientation of the second image sensor 114, bundle adjustment can be used. In some examples, classical bundle adjustment can be used to optimise 3-dimensional structure, the second image sensor pose and intrinsic parameters collectively. In other examples, only the corrective orientation **R**_{*δ*,*m*} and the corrective translation **t**_{*δ*,*m*} are optimised which minimizes the reprojection error: ${min}_{{R}_{δ , m} , {t}_{δ , m}} {\sum }_{m = 0}^{M} {\sum }_{n = 0}^{N} {\left‖d\left(K\left[{R}_{δ , m}\left|{t}_{δ , m}\right.\right]{\hat{T}}_{w \to cam , m}{\dot{x}}_{w , n}\right)-{x}_{ref , n , m}\right‖}_{2}^{2} ,$
where $d \left(K\left[{R}_{δ , m}\left|{t}_{δ , m}\right.\right]{\hat{T}}_{w \to cam , m}{\dot{x}}_{w , n}\right) = \left(\begin{matrix}\frac{{x}_{n , m}}{{z}_{n , m}} \\ \frac{{y}_{n , m}}{{z}_{n , m}}\end{matrix}\right)$ is the dehomogenising operator and
where ${x}_{ref , n , m} = \left(\begin{matrix}{x}_{ref , n , m} \\ {y}_{ref , n , m}\end{matrix}\right)$ is the *n*-th reference point detected in the *m*-th RGB(D) image.

The optimisation problem above can be minimised by a standard numerical optimisation routine such as gradient decent or the like. In some examples, the rotation matrices will be converted to quaternions which are better suited for optimisation.

In examples in which the second image sensor 114 is configured to determine depth in the captured image, the optimization problem can be formulated in a 3-dimensional space. Then, the ICP algorithm can be utilized for the matching of the set of reference points captured in the image to the set of reference points based on system geometry parameters for each system configuration and thus determining the corrective transformations. The optimisation, i.e., the error minimisation, allows the corrective transformations **T**_{*δ*,*m*} = [**R***_{δ,m}*|**t***_{δ,m}*] to be determined for every system configuration *m*. The corrective transformations are the transformations that minimise the reprojection error stated above.

In some examples, the movement of one or more components of the radiographic imaging system 100 between the first configuration and the second configuration is controlled based on the first corrective transformation and the second corrective transformation. For example, the first corrective transformation and the second corrective transformation are applied to control the system parameter of the one or more components of the system 100. The corrective transformation is mapped or transformed into the first reference frame before it is used in the system control. The application of the corrective transformation can be done automatically or manually.

In some examples, at least one of the first and second corrective transformations comprises a transformation applied to at least one system parameter. In other words, the corrective transformations are for changing the system parameter of one or more components of the system 100. The system parameter may represent one or more of an offset parameter of the tube head 112 and the first image sensor 104, a rotation parameter of the tube head 112 and a collimator opening parameter.

The corrective transformation **T***_{δ,m}* can be used to account for system inaccuracies at the system configuration m to compute an error compensated transformation **T**_{*w*→cam,opt,*m*}: ${T}_{w \to cam , opt , m} = {T}_{δ , m} {\hat{T}}_{w \to cam , m}$

Assuming there is provided a movement control instruction, for example, as an outcome of an image-based algorithmic analysis, to move from position 1 to position 2 as **T**_{1→2}, then the error compensated relative motion instruction **T**_{1→2,opt} that is provided to the radiographic imaging system is given by: ${T}_{1 \to 2 , opt} = {T}_{δ , 2} {T}_{1 \to 2} \left({{T}_{δ , 1}}^{- 1}\right) .$

In some examples, a calibration database can be generated. To generate the calibration database, the computer implemented method 300, as shown in Fig. 3 and described hereinbefore, is repeated for various configurations.

To repeat the method 300 to generate a calibration database, steps 312 to 318 are repeated in a plurality of configurations to determine a plurality of corresponding corrective transformations for the plurality of configurations. The repeated steps 312 to 318 are substantially the same as those described hereinbefore for the first configuration. Ideally, to ensure efficient generation of the calibration database, each of the plurality of configurations has at least one system geometry parameter that is different to the corresponding system geometry parameter of the other configurations. The calibration database comprises the plurality of configurations and the corresponding plurality of corrective transformations.

The corrective transformations **T***_{δ,m}* for the calibration database can be generated after the radiographic imaging system is installed. Additionally, the calibration database can be updated by repeating the method described herein, if inaccuracies are observed.

The generated calibration database can be used by the system 100 to correct a system parameter. For example, if the system receives an instruction to change from a current configuration to a new configuration, the corrective transformation corresponding to the new configuration can be determined based on the calibration database. In other words, at runtime, the corrective transformation can be accessed by simple lookup of the closest corrective transformation for the current and the target position. The determined corrective transformation(s) can be applied to control a system parameter.

In an example, if the new configuration is not included in the calibration database, the corrective transformation can be determined by determining a closest configuration to the new configuration comprised in the calibration database, and selecting a corresponding closest corrective transformation as the corrective transformation.

The closest configuration to the new configuration comprised in the calibration database is the configuration that is the most similar to the new configuration, in terms of the system geometry parameters. For example, the closest configuration may be the configuration with the most system geometry parameters in common with the new configuration, or the closeness may be based on the similarity of weighted system geometry parameters, such that an increased similarity in certain system geometry parameters may be more significant in determining the closest configuration than that of other system geometry parameters.

In another example, if the new configuration is not included in the calibration database, the corrective transformation can be determined by determining a first closest configuration to the new configuration comprised in the calibration database, determining a second closest configuration to the new configuration comprised in the calibration database, interpolating between the corrective transformations corresponding to the first closest configuration and the second closest configuration comprised in the database, and generating the corrective transformation based on the interpolation.

The first closest configuration to the new configuration comprised in the calibration database is the configuration that is the most similar to the new configuration, in terms of the system geometry parameters. The second closest configuration to the new configuration comprised in the calibration database is the configuration that is the next most similar to the new configuration, in terms of the system geometry parameters. The closeness of the configurations may be determined by the number of system geometry parameters in common with the new configuration, or the similarity of weighted system geometry parameters, as described hereinbefore.

In some examples, the interpolation is a linear interpolation; however, any suitable interpolation can be used.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer implemented method (300) for controlling a radiographic imaging system (100), wherein the radiographic imaging system comprises an image sensor (114), the method comprising:
determining (310) a first corrective transformation corresponding to a first configuration of the radiographic imaging system by:
(i) obtaining (312) a first set of coordinates corresponding to a set of reference points (132) based on a known geometry of the radiographic imaging system with respect to the set of reference points;
(ii) capturing (314), by the image sensor, an image (134) comprising the set of reference points;
(iii) obtaining (316) a second set of coordinates corresponding to the set of reference points based on image analysis of the captured image;
(iv) determining (318) the corrective transformation by minimising a reprojection error between the first set of coordinates and the second set of coordinates; and
determining (320) a second corrective transformation corresponding to a second configuration of the radiographic imaging system by repeating steps (i) to (iv) for the second configuration, and
wherein the first corrective transformation and the second corrective transformation are for changing a system parameter of one or more components of the radiographic imaging system when in the first configuration or the second configuration.

2. The method of claim 1, further comprising:
controlling a movement of one or more components of the radiographic imaging system between the first configuration and the second configuration based on the first corrective transformation and the second corrective transformation.

3. The method of any preceding claim further comprising:
repeating steps (i) to (iv) for a plurality of configurations of the radiographic imaging system, to determine a plurality of corresponding corrective transformations for the plurality of configurations; and
generating a calibration database comprising the configurations and the corrective transformations.

4. The method of claim 3, further comprising:
receiving an instruction to change the radiographic imaging system from a current configuration to a new configuration;
determining a corrective transformation corresponding to the new configuration based on the calibration database.

5. The method of claim 4, wherein, when the new configuration is not included in the calibration database, the determining the corrective transformation comprises:
determining a closest configuration to the new configuration comprised in the calibration database; and
selecting a corresponding closest corrective transformation as the corrective transformation.

6. The method of claim 4, wherein, when the new configuration is not included in the calibration database, the determining the corrective transformation comprises:
determining a first closest configuration to the new configuration comprised in the calibration database;
determining a second closest configuration to the new configuration comprised in the calibration database; and
interpolating between the corrective transformations corresponding to the first closest configuration and the second closest configuration comprised in the database; and
generating the corrective transformation based on the interpolation.

7. The method of any preceding claim, wherein step (iii) comprises inputting the captured image to a trained machine learning model.

8. The method of any of claims 1 to 6, wherein step (ii) comprises capturing a sequence of images, and step (iv) comprises step (iii) comprises minimising the reprojection error between the first set of coordinates and the second set of coordinates based on bundle adjustment performed on the sequence of images.

9. The method of any preceding claim, wherein the image sensor is a second image sensor, and the plurality of reference points are provided on a first image sensor (104) configured to generate a radiographic image.

10. The method of claim 9, wherein the plurality of reference points comprises at least one of:
a visually distinguishable point of the first image sensor; and
a marker attached to the first image sensor.

11. The method of any preceding claim, wherein the image sensor is further configured to determine depth in a captured image, and the second set of coordinates of the set of reference points comprises three-dimensional position information.

12. The method of any preceding claim, wherein the corrective transformation comprises a transformation applied to at least one parameter representing:
an offset parameter of a tube head (112) of the radiographic imaging system and a first image sensor (104) configured to generate a radiographic image;
a rotation parameter of the tube head; and
a collimator opening parameter of a collimator of the radiographic imaging system.

13. A radiographic imaging system (100) for carrying out the method (300) of any preceding claim comprising:
an image sensor (114) for capturing an image (134) comprising a set of reference points (132); and
a controller (122) configured to carry out the method of any preceding claim.
